# NOUVEAU FASCICULE DE BREVET EUROPEEN

(11) **EP 0 934 743 B2**
(45) Date de publication et mention de la décision concernant l'opposition: **24.11.2010**
(45) Mention de la délivrance du brevet: 02.06.2004
(21) Numéro de dépôt: 99101978.7
(22) Date de dépôt: 01.02.1999
(51) Int. Cl.: A61K 8/04

(54) **Utilisation d'une microdispersion de cire dans une composition cosmétique ou dermatologique**
Verwendung einer Wachsmikrodispersion in der Kosmetik oder Dermatologie
Use of a wax microdispersion in cosmetic or dermatological compositions

(30) Priorité: 09.02.1998 FR 9801494
(43) Date de publication de la demande: 11.08.1999
(73) Titulaire: L'Oréal, 75008 Paris (FR)
(72) Inventeur: Chevalier, Véronique, 94440 Villecresnes (FR); Hurel, Valérie, 91190 Gif sur Yvette (FR)
(74) Mandataire: Kromer, Christophe

(56) Documents cités:
- EP-A- 0 394 078
- EP-A- 0 399 909
- EP-A- 0 446 094
- EP-A- 0 557 196
- WO-A-99/26588
- DE-A1- 3 713 494
- FR-A- 2 666 015
- DATABASE WPI Section Ch, Week 8715 Derwent Publications Ltd., London, GB; Class A96, AN 87-106529 XP002083376 & SU 1 250 297 A (MOSC COSMETICS RES) , 15 août 1986
- DATABASE WPI Section Ch, Week 8815 Derwent Publications Ltd., London, GB; Class D21, AN 88-103905 XP002083377 & SU 1 335 288 A (HETEROORG CPDS NESMEYANK ET AL), 7 septembre 1987

## Description

L' invention se rapporte à l'utilisation d'une microdispersion de cire dans une composition cosmétique ou pour la préparation d'une composition dermatologique à application topique, comme actif destiné à la prévention et/ou au traitement de certains signes du vieillissement endogène et/ou exogène.

Le vieillissement cutané résulte des effets sur la peau de facteurs intrinsèques et extrinsèques. Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat. Sur les zones de la peau qui ont été exposées au soleil tout au long de la vie - essentiellement le visage, le décolleté, les mains et les avant bras - on observe souvent des tâches pigmentaires, des télangiectasies et une élastose.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

Les changements de la peau résultant du vieillissement intrinsèque ou physiologique sont la conséquence d'une sénescence génétiquement programmée où interviennent des facteurs endogènes. Ce vieillissement intrinsèque provoque notamment un ralentissement du renouvellement des cellules de la peau. Histologiquement la peau est globalement amincie, tant au niveau épidermique que dermique. La densité des macromolécules fibreuses du derme (élastine et collagène) est diminuée. Au contraire, le vieillissement extrinsèque entraîne des changements histopathologiques tels qu'une excessive accumulation de matière élastique dans le derme supérieur et une dégénérescence des fibres de collagène.

L'invention s'intéresse essentiellement aux rides, ridules, et à l'éclat de la peau.

On connait de nombreuses compositions qui prétendent traiter les rides et les ridules de la peau ou raffermir les tissus cutanés, mais ces compositions ne traitent qu'incomplétement et temporairement ces désordres morphologiques. Aussi, il subsiste le besoin d'une composition à application topique permettant de traiter plus efficacement les rides et les ridules. L'invention permet, de plus, de donner un éclat à la peau âgée, comparable à celui d'une peau plus jeune.

L'invention a donc pour objet l'utilisation cosmétique d'une microdispersion de cire dont les particules ont des dimensions inférieures à 1µm comme actif destiné à prévenir et/ou traiter l'apparition des rides, des ridules.

L'invention a aussi pour objet un procédé cosmétique de ravivement de l'éclat de la peau par traitement des rides et/ou des ridules en utilisant une microdispersion de ciré dont les particules ont des dimensions inférieures à 1 µm, appliquée sur la peau dans une composition cosmétique.

On connaît, par le document SU-1335288, l'utilisation de cires émulsionnées dans des compositions destinées au traitement du relâchement de la peau. Toutefois, dans les compositions décrites dans ce document, les cires émulsionnées sont utilisées comme agent émulsionnant, leur utilisation comme actif pour prévenir et/ou traiter l'apparition des signes du vieillissement cutané n'est ni mentionnée, ni suggérée par ce document.

On sait également, en particulier par le document SU-1250297, que les cires peuvent être utilisées dans le traitement des peaux âgées, en particulier des rides. Toutefois, la demanderesse a découvert que, de façon inattendue, les microdispersions de cire présentaient une efficacité bien supérieure à celle des cires classiques dans la prévention et le traitement des signes du vieillissement cutané.

Les microdispersions de cire, qui sont des dispersions stables de particules colloïdales de cire, sont connues et peuvent être préparées selon des méthodes connues ; voir par exemple "Microemulsions Theory and Practice", L.M. Prince Ed., Academic Press (1977) pages 21-32.

Les particules de la microdispersion de cire ont des dimensions inférieures à 7 µm, de préférence inférieures à 0,5 µm. Ces particules sont constituées essentiellement d'une cire ou d'un mélange de cires. Le point de fusion de la cire ou, du mélange de cires est de préférence compris entre 50°C et 150°C. En outre, les particules de la microdispersion peuvent contenir en proportion minoritaire des additifs gras huileux ou pâteux, un ou plusieurs tensioactifs et un ou plusieurs ingrédients actifs liposolubles usuels, comme cela sera précisé ci-après.

La composition contient généralement de 0,1 à 40 % en poids de cires, en particulier 5 à 30 %, et une quantité suffisante d'au moins un émulsionnant. La quantité d'émulsionnant est une quantité suffisante pour permettre d'obtenir une microdispersion de cire telle que définie ci-dessus. Cette quantité suffisante peut être déterminée dans chaque cas par des expériences de routine.

Les cires sont des substances naturelles (animales ou végétales) ou synthétiques solides à température ambiante (20°-25°C). Elles sont insolubles dans l'eau, solubles dans les huiles et sont capables de former un film hydrofuge.

Sur la définition des cires, on peut citer par exemple P.D. Dorgan, Drug and Cosmetic Industry, Decembre 1983, pp. 30-33.

La cire ou les cires constituant le mélange cireux sont choisies notamment, parmi la cire de Carnauba, la cire de Candelila, et la cire d'Alfa, et leurs mélanges.

Outre les cires citées ci-dessus, le mélange de cires peut également contenir une ou plusieurs des cires ou famille de cires suivantes :
- la cire de paraffine
- l'ozokérite
- les cires végétales comme la cire d'olivier, la cire de riz, la cire de jojoba hydrogénée ou les cires absolues de fleurs telles que la cire essentielle de fleur de cassis vendue par la Société BERTIN (France).
- les cires animales comme les cires d'abeilles, ou les cires d'abeilles modifiées (cerabellina) ;
- d'autres cires ou matières premières cireuses : les cires marines telles que celle vendue par la Société SOPHIM sous la référence M82, les céramides naturels ou de synthèse, ou les cires de polyéthylène ou de polyoléfines en général.

Les cires végétales de Carnauba (extraite de Copernica Cerifera), de Candelilla (extraite de Euphobies Cerifera et de Pedilantus pavonis) et d'Alfa (extraite de Stipa tenacissima), sont des produits commerciaux.

Les céramides sont les principaux lipides constitutifs des espaces intercoméocytaires du Stratum Comeum. Ils sont décrits en particulier par Downing dans Science, 1982, vol. 18, P. 1261-1262. Des analogues synthétiques sont également connus, tels que les céramides HO3 vendus par la Société COSMIND.

Dans le mélange de cires, la cire de Carnauba et/ou de Candelilla et/ou d'Alfa représente au moins 20 %, et de préférence au moins 50 % en poids par rapport au poids total du mélange de cires.

La cire ou le mélange de cires peut contenir, outre les cires mentionnées ci-dessus, au moins une autre cire et/ou au moins une huile, étant entendu que le mélange de cires et éventuellement d'huile a un point de fusion finissante supérieur à 50°C.

Le mélange de cires peut donc être associé à un ou plusieurs additifs gras (huileux ou pâteux). On citera de manière non restrictive :
- les huiles végétales comme l'huile de tournesol, l'huile dejojoba, etc.;
- les huiles minérales comme l'huile de paraffine ;
- les huiles de silicones fluides de viscosité comprise notamment entre 0.65 et 100.000 centistokes (soit entre 0,65.10⁻⁴ et 10m².s⁻¹, de préférence entre 5 et 5000 centistokes (soit entre 5.10⁻⁴ et 5.10⁻¹m².s⁻¹) ;
- les huiles fluorées ;
- la vaseline ;
- la lanoline.

Le mélange d'huile(s) et/ou d'additifs gras pâteux peut représenter jusqu'à 30 %, de préférence au plus 10 %, du poids de cires.

Il est possible d'introduire en outre dans la phase cireuse microparticulaire des ingrédients actifs liposolubles.

Lorsqu'ils sont présents, le ou les ingrédients actifs liposolubles représentent au maximum 30 %, de préférence au maximum 10 %, du poids des microparticules.

L'utilisation de tensioactifs comme émulsionnants dans la préparation de microdispersions de cires est connue. La réalisation de la microdispersion peut être effectuée à l'aide de tensioactifs anioniques, cationiques et/ou non ioniques, de façon connue.

Le pourcentage de tensio-actif(s) dans la composition finale est compris généralement entre 0.01 et 25 % environ et en particulier peut varier de 0,1 à 10 %.

Le rapport pondéral cire(s)/émulsionnant(s) peut varier par exemple dans la gamme de 1 à 30 et notamment de 2 à 10.

Les tensioactifs anioniques utilisés sont notamment les sels d'acides gras (par exemple sels alcalins ou sels organiques tels que les sels d'amines) lesdits acides gras ayant par exemple de 12 à 16 atomes de carbone et pouvant comporter une double liaison comme dans le cas de l'acide oléique, les sels alcalins ou sels de bases organiques des acides alkyl-sulfuriques et alkylsulfoniques ayant 12 à 18 atomes de carbone, des acides alkyl-arylsulfoniques dont la chaîne alkyle contient de 6 à 18 atomes de carbone, le groupement aryle étant par exemple un groupement phényle. Ce sont également les éthers-sulfates, en particulier les produits de sulfatation des alcools gras alkylphénols polyalcoxylés dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Tous ces tensioactifs anioniques sont bien connus et beaucoup d'entre eux sont des produits commerciaux.

Les tensio-actifs non ioniques sont principalement des tensio-actifs polyalcoxylés et/ou polyglycérolés. Ce sont notamment les acides gras ou les amides d'acide gras polyalcoxylés et:ou polyglycérolés ; les alcools gras ou les alkylphénols polyalcoxylés et/ou polyglycérolés ; les esters d'acides gras et de polyols polyalcoxylés et/ou polyglycérolés ; les alcanediols ou alcènediols-1,2 ou -1,3 polyalcoxylés et/ou polyglycérolés : et les alkyléthers d' alcanediols ou alcènediols-1,2 ou -1,3 polyalcoxylés et/ou polyglycérolés. Par exemple, les acides ou alcools gras, éventuellement insaturés, ont 12 à 24 atomes de carbone, la chaîne alkyle des alkylphénols a 6 à 16 atomes de carbone, les alcanediols ou alcènediols ont de 9 à 24 atomes de carbone, l'alkyle des alkyléthers a de 4 à 20 atomes de carbone, et le nombre de motifs oxyalkylène ou de motifs (CH₂CHOHCH₂O) peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la marque Myrj par la société ATLAS.

Les esters d'acide gras et de polyols polyoxyéthylénés pour lesquels le polyol est le sorbitol sont des produits connus (Polysorbate et produits commercialisés sous la marque Tween par la Société ATLAS). Lorsque le polyol est le glycérol, on peut utiliser les produits commercialisés sous la marque Brij par la société ATLAS.

Les alcools gras polyglycérolés, les alcanediols ou alcanediols polyglycérolés, ou les alkyéthers d'alcanediols ou d'alcènediols polyglycérolés peuvent être préparés par exemple selon les procédés décrits dans les brevets français 1.477.048, 2.025.681, 2.091.516 et 2 .465 .780 ou selon des procédés analogues.

Les acides gras ou amides d'acides gras polyglycérolés sont notamment décrits dans le brevet français 1.484.723 ou sont encore des produits commerciaux tels que ceux vendus sous la marque PLUROL (Gattefossé) ou DREWPOL, (Stefan Company), ou DECAGLYN (Nikko Chemical).

D'autres tensioactifs non-ioniques utilisables sont par exemple :
- les alkylcarbamates de triglycérol de formule générale :

   R-NHCOOCH(CH₂OCH₂CHOHCH₂OH)₂

   dans laquelle R représente un groupe alkyle saturé ou non de 10 à 20 atomes de carbone. Ces composés sont décrits dans le brevet EP 0420761 ;
- les dérivés oxyéthylénés ou propoxylés des alcools de la lanoline, des acides gras de la lanoline, ou de leur mélanges.

De tels tensioactifs sont commercialisés par la Société AMERCHOL sous la marque SOLULAN.

Les tensioactifs anioniques sont notamment les dérivés d'ammonium quaternaire tels que l'ARQUAD 16-50, l'ARQUAD 18-50, l'ARQUAD T-50, l'ARQUAD 2C-75, l'ETHOAQUAD c/12, et l'ETHOQUAD o/12, commercialisés par la Société Armak Chemicals.

L'emploi des tensioactifs non ioniques est préféré.

Il est également possible de préparer des microdispersions de cires en utilisant des mélanges commerciaux de cires auto-émulsionnables contenant la cire et les tensioactifs.

On peut utiliser par exemple la cire commercialisée sous la dénomination CIRE AUTO LUSTRANTE OFR par la Société TISCCO, qui contient des cires de Carnauba et de paraffine, en association avec des agents émulsionnants non ioniques, ou la cire auto-émulsionnable commercialisée sous la dénomination CERAX A.O. 28/B par LA CERESINE, qui contient de la cire d'Alfa en association avec un émulsionnant non ionique.

Ces mélanges commerciaux permettent de préparer des microdispersions de cires par addition d'eau selon le procédé décrit ci-dessus.

On peut également utiliser des microdispersions de cires prêtes à l'emploi disponibles commercialement comme les produits de la série SLIP-AID de la Société DANIEL PRODUCTS COMPANY, ou encore les produits Aquacer de la société CERACHEMIE.

Les microdispersions de cires sont diluables à l'eau sans nuire à la stabilité de la microdispersion. Elles peuvent donc se présenter sous la forme de compositions concentrées dont on peut ajuster la proportion des ingrédients à une valeur désirée par simple addition d'eau.

La composition contient un milieu cosmétiquement ou dermatologiquement acceptable, c'est-à-dire compatible avec les tissus cutanés. Ainsi, la composition peut être appliquée sur tout le corps humain.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs lipophiles ou hydrophiles utilisables dans l'invention en vue de parfaire le traitement des rides, ridules, la lutte contre le relâchement cutané et/ou sous-cutané et/ou l'éclat de la peau, on peut citer par exemple les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les α-hydroxy-acides (glycolique, lactique, malique, citrique, tartrique, mandélique), les β-hydroxy-acides (acide salicylique et ses dérivés notamment alcoylés), les α-céto-acides, les β-céto-acides, les peroxydes comme le peroxyde de benzoyle, les vitamines notamment E, F, les actifs anti-radicaux comme la superoxyde-dismutase, le sélénium, le zinc, les béta-carotènes, les polymères tenseurs d'origine naturelle ou synthétique.

La composition peut en outre contenir des hormones naturelles ou synthétiques, oestrogéniques, progestatives ou androgéniques comme la progestérone, la testostérone, l'oestradiol anhydre, le broparestrol, l'oestrone, l'acétate de prégnénolone, la prégnénolone, la 17 béta-hydroxy- progestérone, le propionate de testostérone, l'androstènedione et les androstanediols.

Les compositions selon l'invention sont obtenues par formation à chaud d'une microémulsion. Plus précisément, ces compositions sont obtenues par un procédé principalement caractérisé par le fait que l'on chauffe la cire et l'émulsionnant à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microémulsion de cire dans une phase continue aqueuse, puis que l'on laisse refroidir jusqu'à la température ambiante. On obtient une microdispersion stable de cire.

On opère avec une agitation et une quantité de tensio-actif suffisantes pour que les dimensions de microparticules de cire soient inférieures à 1 000 nm, et de préférence à 500 nm.

Les ingrédients liposolubles, par exemples des céramides, sont généralement ajoutés à la cire avant la réalisation de la microdispersion.

Les ingrédients hydrosolubles peuvent être ajoutés dans l'eau utilisée pour réaliser la microdispersion, ou dans la microdispersion de cire finalement obtenue.

De même, les ingrédients secondaires éventuellement présents dans la composition sont ajoutés selon les cas soit dans les produits de départ, soit dans la composition terminée.

Les exemples suivants sont donnés à titre d'illustration de l'invention. Les pourcentages sont donnés en poids de matière première par rapport au poids total de la composition. Les noms des constituants sont donnés en nom INCI.

### Exemple 1 : Crème anti-rides (émulsion eau-dans-huile)

| Phase A : | |
|---|---|
| Hydrogenated polyisobutene | 5,5 % |
| Isostearyl neopentanoate | 3,5 % |
| PEG-20 stearate | 1 % |
| Glyceryl stearate et PEG-100 stearate | 2 % |
| Cetyl alcohol | 0,5 % |
| Stearyl alcohol | 0,5 % |
| Stearic acid | 3 % |

| Phase A' : | |
|---|---|
| Cyclomethicone | 11 % |

| Phase C : | |
|---|---|
| Polyacrylamide et C13-C14 isoparaffin et laureth-7 | 1,7 % |

| Phase D : | |
|---|---|
| Polyethylene (AQUACER 513 commercialisée par la société CERACHIMIE) | 20 % |

| Phase B : | |
|---|---|
| Conservateurs | qs |
| Sodium hydroxyde | 0,03 % |
| Eau | qsp 100 % |

### Mode opératoire :

On chauffe la phase A sous agitation jusqu'à homogénéité. Après refroidissement on ajoute la phase A'. On chauffe la phase B sous agitation, puis on verse B dans A toujours sous agitation. Après refroidissement à 50°C on incorpore à l'émulsion la phase C, puis à 40°C, la phase D.

### Exemple 2 : Sérum anti-vieillissement

| | |
|---|---|
| Polyacrylamide et C13-C14 isoparaffin et laureth-7 | 1 % |
| Xanthan gum | 0,2 % |
| PVM/MA Decadiene Crosspolymer | 0,2 % |
| Triethanolamine | 0,2 % |
| Polyethylene (AQUACER 513 commercialisée par la société CERACHIMIE) | 10 % |
| Conservateur | qs |
| Eau | qsp 100 % |

Mode opératoire : On disperse la gomme de xanthane et le polymère (PVM/MA Decadiene Crosspolymer) dans l'eau à chaud sous agitation avec les conservateurs et la triéthanolamine. On refroidit à 40°C et on incorpore la microcire (polyéthylène) et le polyacrylamide, toujours sous agitation.

## Revendications

1. Utilisation cosmétique d'une microdispersion de cire dont les particules ont des dimensions inférieures à 1 µm, comme actif destiné à prévenir et/ou traiter l'apparition des rides et/ou des ridules.

2. Utilisation selon la revendication 1 , **caractérisée en ce que** les particules de la microdispersion de cire ont des dimensions inférieures ou égales à 0,5 µm.

3. Utilisation selon l'une quelconque des revendications 1 à 2 précédentes, **caractérisée en ce que** les particules de la microdispersion de cire sont constituées essentiellement d'une cire ou d'un mélange de cires dont le point de fusion est compris entre 50°C et 150°C

4. Procédé cosmétique de ravivement de l'éclat de la peau par traitement des rides et/ou des ridules en utilisant une microdispersion de cire dont les particules ont' des dimensions inférieurs à 1 µm, appliquée sur la peau dans une composition cosmétique.

5. Procédé selon la revendication 4, **caractérisé en ce que** la composition contient de 0,1 à 40 % en poids de cires, en particulier 5 à 30 %.

## Claims

1. Cosmetic use of a microdispersion of wax in which the particles are less than 1 µm in size, as an active agent intended to prevent and/or treat the appearance of wrinkles and/or fine lines.

2. Use according to Claim 1, **characterized in that** the particles in the microdispersion of wax are less than or equal to 0.5 µm in size.

3. Use according to either one of Claims 1 and 2 above, **characterized in that** the particles in the microdispersion of wax consist essentially of a wax or of a mixture of waxes whose melting point is between 50°C and 150°C.

4. Cosmetic process for reviving the radiance of the skin by treating wrinkles and/or fine lines while using a microdispersion of wax in which the particles are less than 1 µm in size, which is applied to the skin in a cosmetic composition.

5. Process according to Claim 4, **characterized in that** the composition contains from 0.1 to 40% by weight of waxes, in particular 5 to 30%.

## Patentansprüche

1. Kosmetische Verwendung einer Mikrodispersion von Wachs, deren Partikel Abmessungen unter 1 µm aufweisen, als Wirkstoff, der zur Vorbeugung und/ oder zur Behandlung des Auftretens von Falten und/oder Fältchen bestimmt ist.

2. Verwendung nach Anspruch 1, **dadurch gekennzeichnet, dass** die Partikel der Mikrodispersion von Wachs Abmessungen von kleiner oder gleich 0,5 µm aufweisen.

3. Verwendung nach einem der vorhergehenden Ansprüche 1 bis 2, **dadurch gekennzeichnet, dass** die Partikel der Mikrodispersion von Wachs im Wesentlichen aus einem Wachs oder einem Gemisch von Wachsen bestehen, deren Schmelzpunkt im Bereich von 50 bis 150 °C liegt.

4. Kosmetisches Verfahren zum Auffrischen des Teints der Haut durch Behandlung von Falten und/oder Fältchen unter Verwendung einer Mikrodispersion von Wachs, deren Partikel Abmessungen von kleiner 1 µm aufweisen und die in einer kosmetischen Zusammensetzung auf die Haut aufgebracht wird.

5. Verfahren nach Anspruch 4, **dadurch gekennzeichnet, dass** die Zusammensetzung 0,1 bis 40 Gew.-% Wachse und insbesondere 5 bis 30 Gew.-% Wachse enthält.
